# EUROPÄISCHE PATENTSCHRIFT

(11) **EP 0 827 415 B1**
(45) Veröffentlichungstag und Bekanntmachung des Hinweises auf die Patenterteilung: **06.03.2002**
(21) Anmeldenummer: 96914961.6
(22) Anmeldetag: 19.04.1996
(51) Int. Cl.: A61M 1/36

(54) **VORRICHTUNG ZUR GEFÄSSDRUCKGESTEUERTEN SELEKTIVEN PERFUSION VON KÖRPERGEFÄSSEN MIT FLUIDEN**
DEVICE FOR THE VASCULAR PRESSURE-CONTROLLED SELECTIVE PERFUSION OF FLUIDS THROUGH BLOOD VESSELS
DISPOSITIF PERMETTANT D'INJECTER DES FLUIDES DANS DES VAISSEAUX SANGUINS PAR PERFUSION SELECTIVE REGULEE PAR LA PRESSION VASCULAIRE

(30) Priorität: 20.04.1995 DE 19514638
(43) Veröffentlichungstag der Anmeldung: 11.03.1998
(73) Patentinhaber: Boekstegers, Peter, 86911 Diessen (DE)
(72) Erfinder: Boekstegers, Peter, 86911 Diessen (DE)
(74) Vertreter: Kinzebach, Werner, Dr.
(86) Internationale Anmeldenummer: EP9601657
(87) Internationale Veröffentlichungsnummer: WO9632972

(56) Entgegenhaltungen:
- EP-A- 0 357 338
- EP-A- 0 364 799
- WO-A-95/23620
- US-A- 5 024 668
- CARDIOVACULAR RESEARCH, Nr. 24, 1990, Seiten 456-464, XP000577523 P.BOEKSTEGERS: "selective ECG synchronised suction and retroinfusion of coronary veins:first results studies in acute myocardial ischaemia in dogs" in der Anmeldung erwähnt
- IEEE TRANSACTIONS ON BIO-MEDICAL ENGINEERING, Bd. 11, Nr. 3, Juli 1964, Seiten 94-102, XP002009452 H SHIRER: "a servo-controlled,whole body,blood perfusion system as a pressure/flow clamp"

## Beschreibung

Die vorliegende Erfindung betrifft eine Vorrichtung zur gefäßdruckgesteuerten selektiven Perfusion von Körpergefäßen mit Fluiden. Inbesondere betrifft die vorliegende Erfindung einerseits die venendruckgesteuerte Absaugung und Retroinfusion eines Fluids aus bzw. in Körpervenen, insbesondere Koronarvenen, und andererseits die arteriendruckgesteuerte Perfusion von Körperarterien, insbesondere Koronararterien.

Die nutritive Perfusion von Koronararterien bzw. die Retroinfusion von Blut in Koronarvenen spielt insbesondere im Bereich der Myokardprotektion während eines kurzfristigen Koronararterienverschlusses im Rahmen eines kardiologischen Eingriffs eine zunehmend wichtiger werdende Rolle. Ein typischer derartiger Eingriff ist beispielsweise die Ballondilatation einer arteriosklerotisch verengten Koronararterie. Bei dieser auch als perkutane transluminale Koronarangioplastie (PTCA) bekannten Methode wird ein Ballonkatheter unter Röntgenkontrolle in den Bereich der Stenose der Koronararterie geführt und die arteriosklerotische Plaque durch Aufblasen des am Ende des Katheters befindlichen Ballons komprimiert. Während der Dilatation des Ballons findet stromabwärts in der Arterie keine Versorgung des Gewebes mit sauerstoffhaltigem Blut statt. Dies stellt meist kein Problem dar, solange die Dilatation nur kurzzeitig erfolgt. Bei Dilatationen bereits ab 30 Sekunden Dauer lassen sich allerdings funktionelle Veränderungen im Ischämiegebiet des Myokard feststellen, so zum Beispiel elektrokardiographisch als S-T-Veränderungen, echokardiographisch als verminderte regionale Wandbewegung oder auch subjektiv als Angina pectoris Beschwerden des Patienten. Darüber hinaus ist das Komplikationsrisiko bei einer Angioplastie für bestimmte Patientengruppen erhöht, etwa bei älteren Patienten, bei Vorliegen einer instabilen Angina pectoris, bei erniedrigter linksventrikulärer Auswurffraktion oder bei Dilatation eines Gefäßes, das mehr als 40% des linken Ventrikels versorgt.

Entsprechende Probleme der Ischämieprotektion des Myokard stellen sich auch bei anderen Eingriffen zur Koronarvaskularisierung wie z.B. bei Atherektomie, Koronarendoprothesen und Laseranwendungen.

Es ist bekannt, eine kurzzeitigen Ischämieprotektion durch maschinelle Perfusion einer die betroffen Myokardregion versorgenden Arterie, etwa der zu dilatierenden Arterie selbst, entweder mit arteriellem Blut, das an anderer Stelle dem Patienten entnommen wurde, oder mit anderen nutritiven Fluiden durchzuführen. Hierbei besteht jedoch die Gefahr, daß es zu einer Überperfusion des Myokardgewebes kommt, was insbeondere dann der Fall ist, wenn der Abstrom des perfundierten Fluids aus dem betroffenen Gewebe behindert oder vollständig blockiert ist. In derartigen Fällen kann es zu hämorrhagischen Gewebeinfarkten in der betroffenen Myokardegion kommen.

Als eine weitere Möglichkeit zur kurzzeitigen Ischämieprotektion wird in Fällen, in denen Komplikationen zu erwarten sind, seit einiger Zeit eine Retroinfusion von arteriellem Blut in eine Vene des betreffenden Ischämiegebiets des Myokards durchgeführt. Das arterielle Blut wird dabei über die entsprechende Vene in die nutritiven Kapillaren des Ischämiegebietes gepumpt und versorgt so das Myokard in dieser Region mit Sauerstoff und Substraten.

Vorrichtungen zur Retroinfusion von Koronarvenen sind seit einigen Jahren bekannt. So ist in der europäischen Patentschrift EP-B-0 297 723 ein Retroinfusionsgerät beschrieben, mit dem arterielles Blut, beispielsweise aus der Arteria femoralis dem Patienten entnommen und über ein Pumpsystem und einen aufblasbaren Ballonkatheter einer Koronarvene des Ischämiegebiets zugeführt wird. Das Pumpen von arteriellem Blut in die Koronarvene wird dabei mit der R-Welle eines Elektrokardiogramms des Patienten synchronisiert, so daß das Pumpintervall dem Herzzyklus des Patienten angepaßt ist. Dabei ist das Pumpintervall fest vorgegeben und beginnt bei 45% des R-R-Intervalls und endet bei 95% des R-R-Intervalls. Der infundierte Blutfluß ist dabei während des Pumpintervalls im wesentlichen konstant. Solange gepumpt wird, ist der Ballon des Ballonkatheters aufgeblasen und blockiert die Vene, so daß sichergestellt ist, daß arterielles Blut während der Diastole effektiv in das Ischämiegebiet transportiert wird. Der Pumpvorgang endet mit dem Ende der Diastole und der Ballon wird entleert, so daß der Fluß in der Vene an dieser Stelle nicht mehr blockiert ist. Während der darauffolgenden Systole kann venöses Blut über die Vene abströmen.

Mit der in der EP 0 297 723 beschriebenen Vorrichtung läßt sich der Basisstoffwechsel des Ischämiegebiets während eines kurzzeitigen kardiologischen Eingriffs zufriedenstellend aufrechterhalten. Ferner konnte beobachtet werden, daß die Myokardinfarktgröße nach einem Koronararterienverschluß deutlich reduziert wurde. Allerdings wurde auch festgestellt, daß die lokale Myokardfunktion nicht ausreichend aufrechterhalten wird. Beispielsweise kommt die lokale Myokardfunktion bei fehlender arterieller Kollateralisierung des Ischämiegebiets vollständig zum Erliegen. Als Ursache hierfür ist in erster Linie der unvollständige Austausch von arteriellem und venösem Blut in dem retroinfundierten Venensystem im Laufe eines Herzzyklusses zu sehen.

Zur Verbesserung dieses Blutaustauschs mit dem Ziel, eine verbesserte Aufrechterhaltung der Myokardfunktion im Ischämiegebiet zu erreichen, haben Boekstegers et al kürzlich in *Cardiovascular Research* 1990, **24**: 456-464 und in *JACC* 1994, **23**: 459-469 ein System zur Retroinfusion von Koronarvenen vorgeschlagen, bei dem anstelle des passiven Abströmens von venösem Blut während der Systole ein aktives Absaugen durch den Retroinfusionskatheter stattfindet. Dazu bleibt der Ballon des Katheters auch im Absaugintervall aufgeblasen und blockiert die Vene auch während der Systole. Mit einer derartigen Vorrichtung wurde im Tiermodell eine bessere Aufrechterhaltung der Myokardfunktion während eines Koronararterienverschlusses nachgewiesen.

Für einen klinischen Einsatz am Patienten weist das System jedoch noch Nachteile auf.

So ist bei dieser Vorrichtung, wie auch bei der Vorrichtung der EP 0 297 723 zwar das Pumpvolumen pro Pumpstoß einstellbar, der Fluß des retroinfundierten Blutes während eines Pumpintervalls kann jedoch nicht beinflußt werden. Es zeigt sich jedoch, daß dadurch der intravenöse Druck im Laufe eines Pumpintervalls starken Schwankungen unterliegt. Selbst wenn man den intravenösen Druck registriert, kann man zwar im Laufe mehrerer Pumpintervalle einen gewünschten mittleren Druck einstellen, die starken Druckschwankungen innerhalb eines Pumpintervalls lassen sich jedoch nicht beseitigen.

Mit diesen starken Änderungen des Koronarvenendrucks sind jedoch einige Probleme verbunden. So wurde festgestellt, daß mit einem niedrigen Retroinfusionsfluß, d.h. bei niedrigem Venendruck, eine ausreichende Versorgung des Myokards mit Sauerstoff nicht gewährleistet ist, so daß die Myokardfunktion im Ischämiegebiet nicht aufrechterhalten werden kann. Bei hohem Infusionsfluß, d.h. bei einem zu hohen Koronarvenendruck besteht jedoch die Gefahr, daß eine Überperfusion auftritt, welche die retrograde nutritive Kapillarfüllung nicht verbessert, sondern nur die Kontraktion des Myokard behindert und zu einem ineffektiven Abstrom des arteriellen Blutes in die systematische Zirkulation führt. Bei Retroinfusion mit einem zu hohen Koronarvenendruck besteht außerdem die Gefahr von irreversiblen Gefäßwandschädigungen.

Schließlich ist aus EP-A-0 364 799 eine Vorrichtung zum Abtransport von Blut aus einem extrakorporalen Kreislauf bekannt, die eine in ein Körpergefäß eines Patienten einbringbare, am proximalen Ende offene Schlauchleitung aufweist, wobei am proximalen Ende ein vergößerbares Dichtmittel vorgesehen ist, mit dem das Gefäß gegenüber der Leitung abdichtbar ist. Außerdem sind Mittel zur Messung des Gefäßinnendrucks vorgesehen.

Aufgabe der vorliegenden Erfindung ist es daher, eine Vorrichtung zur Perfusion von Körpergefäßen, insbesondere von Koronargefäßen anzugeben, bei der die Perfusion bei einem für die nutritive Kapillarfüllung optimalen Gefäßdruck durchgeführt wird und wobei dieser Solldruck beim Pumpen möglichst konstant gehalten wird. Dabei soll die erfindungsgemäße Vorrichtung es ermöglichen, die Einsatzmöglichkeiten der Gefäßperfusion, insbesondere der Arterienperfusion und der Venenretroinfusion, über die bisher durchgeführte kurzfristige Myokardprotektion hinaus zu erweitern.

Gelöst wird diese Aufgabe durch die Vorrichtung gemäß Anspruch 1, deren Anwendung im folgenden beschrieben wird: Man führt eine am proximalen Ende offene Schlauchleitung für die Perfusion einer Geweberegion mit Fluiden in ein zu perfundierendes Körpergefäß eines Patienten ein, dichtet das Gefäß gegenüber der Leitung im Bereich von dessen proximalem Ende ab und pumpt das Fluid in das Gefäß, wobei man einen bestimmten Sollwert des Gefäßinnendrucks vorgibt, den Innendruck des Gefäßes mißt, und den perfundierten Fluidfluß während des Pumpens so regelt, daß der Sollwert des Gefäßinnendrucks möglichst genau eingehalten wird.

Im Gegensatz zu den bekannten Perfusionsverfahren ist erfindungsgemäß der Fluidfluß während des Pumpens von Fluid nicht mehr nur durch das Pumpvolumen pro Pumpstoß bestimmt und damit der Volumenflußverlauf während der Pumphase nicht beeinflußbar, sondern er wird dem gemessenen Gefäßdruck entsprechend reguliert. Als Regulationsverfahren können allgemeine, in der Meß- und Regelungstechnik bekannte Verfahren zum Einsatz kommen, wobei der aktuelle Blutdruck in dem Gefäß als Regelgröße und der aktuell infundierte Fluidfluß als Stellgröße herangezogen werden. Die Regelfunktion kann dabei die typischen elastischen Eigenschaften eines Blutgefäßes anhand vorgegebener Parameter berücksichtigen und diese Parameter gegebenenfalls durch Auswertung der Dynamik des Systems an die im spezifischen Einzelfall vorliegenden Gegebenheiten anpassen. Insbesondere wird das Regelsystem aufgrund des aktuellen Druckverlaufs den zukünftigen Druckverlauf extrapolieren und den Fluidfluß rechtzeitig anpassen. Das Regelsystem wird so eingestellt, daß die Ansprechzeit weniger als 25 Millisekunden beträgt.

Vorteilhaft pumpt man das Fluid periodisch in Intervallen in das Gefäß, wobei man die Pumpintervalle mit dem Herzschlag des Patienten synchronisiert.

Man kann jedoch, insbesondere im Fall der Perfusion von Arterien, das Fluid auch kontinuierlich in das Gefäß pumpen.

Der einzustellende Sollwert des Gefäßdrucks ist ein individueller Wert, der vom jeweiligen Patienten, dem zu infundierenden Gefäß und dem spezifischen Ort der Perfusion in dem Gefäß abhängig ist.

Den Sollwert des Arterieninnendrucks wählt man vorteilhaft so, daß die nutritive Perfusion aufrechterhalten wird.

Wird nutritives Fluid in eine Vene retroinfondiert, so infundiert man periodisch das Fluid über die Schlauchleitung in die Vene infundiert und saugt Blut über die Schlauchleitung aus der Vene ab. Bevorzugt werden die Pump- und Saugintervalle mit dem Herzschlag des Patienten synchronisiert.

Überraschend wurde gefunden, daß es möglich ist, den gewünschten Sollwert des Venendrucks in einer separaten Messung vor Beginn der eigentlichen Retroinfusion für die jeweilige Situation individuell festzulegen. Es wird der Sollwert des Veneninnendrucks so bestimmt, daß man bei abgedichteter Vene entweder ohne Retroinfusion bei vorhandenem venösem Blutfluß den Venendruck mißt, oder daß man bei fehlendem Blutfluß einen mit jedem Pumpintervall ansteigenden Fluidfluß infundiert und dabei den Veneninnendruck mißt. Man stellt fest, daß der Venenspitzendruck nicht proportional zum ansteigenden Fluidfluß zunimmt, sondern sich vielmehr einem Grenzwert (Plateaudruck) nähert. Es ist zu vermuten, daß bei diesem Plateaudruck die maximale retrograde nutritive Kapillarfüllung erreicht ist, und daß bei höherem Fluidfluß lediglich ein ineffektiver Abstrom in die systematische Zirkulation stattfindet. Es wird daher vorgeschlagen, diesen Plateaudruck als Sollwert für den Venendruck bei der Retroinfusion vorzugeben.

Im Fall der Ischämieprotektion des Myokards oder der Gewebeprotektion allgemein ist das infundierte oder perfundierte Fluid bevorzugt ein Sauerstoffträger. Vorteilhaft verwendet man als Sauerstoffträger Blut, wobei besonders vorteilhaft arterielles Blut des Patienten selbst verwendet wird, das beispielsweise aus der Arteria femoralis entnommen und Über eine Pumpe, einem Blutfilter zur Reinigung und einer Luftfalle zur Befreiung des Blutes von Luftblasen unter einem Druck von bevorzugt ca. 2 Bar über eine Durchflußkontrolle der Vene, die retroinfundiert werden soll, zugeführt wird. Es ist aber auch möglich, einen Blutersatzstoff, wie z.B. eine Fluorcarbon- oder Perfluorcetylbromid-Lösung als Sauerstoffträger zu verwenden.

Das dem Patienten venös infundierte oder arteriell perfundierte Fluid kann aber auch therapeutische oder diagnostische Wirkstoffe, wie z.B. Gerinnungshemmer, Kontrastmittel oder β-Blocker enthalten.

Handelt es sich bei der zu retroinfundierenden Körpervene beispielsweise um eine Beinvene, in der sich ein Thrombus befindet, so wird man dem Fluid vorteilhaft Mittel zur Auflösung dieses Thrombus zugeben. Damit lassen sich beispielsweise hohe Konzentrationen eines Medikaments lokal applizieren, ohne daß die übrigen Körperfunktionen davon nachteilig beeinflußt werden.

Gemäß einer Ausführungsform wird venöses Blut bzw. Retroinfusat zwischen den Pumpphasen aktiv abgesaugt. Dieses Blut wird über eine Unterdruckpumpe in ein Reservoir geleitet. Bei einer kurzzeitigen Retroinfusion einer Körpervene ist die so abgesaugte Blutmenge relativ gering. Bei einer längerfristigen Retroinfusion, beispielsweise bei einer Ischämieprotektion des Myokard während eines längerfristigen Koronararterienverschlusses, kann es vorteilhaft sein, das in einem Saugintervall abgesaugte Blut zu entschäumen und von Luftblasen zu befreien und es dann dem Patienten über eine Körpervene wieder zuzuführen. Damit wird ein, die Anwendungsdauer des Verfahrens begrenzender Blutverlust des Patienten wirkungsvoll vermieden.

Erfindungsgemäß wird eine Vorrichtung zur gefäßdruckgesteuerten selektiven Perfusion von Körpergefäßen mit einem Fluid, die insbesondere zur Durchführung des oben beschriebenen Verfahrens geeignet ist, bereitgestellt. Die Vorrichtung weist eine in ein Körpergefäß eines Patienten einbringbaren, am proximalen Ende offenen Schlauchleitung auf, die mit einem unter Druck stehenden Fluid beschickbar ist, das in das Körpergefäß gepumpt werden soll, wobei am proximalen Ende ein vergrößerbares Dichtungsmittel vorgesehen ist, mit dem das Gefäß gegenüber der Leitung abdichtbar ist. Außerdem sind Mittel zur Messung des Gefäßinnendrucks und Regelungsmittel vorgesehen, mit deren Hilfe eine Steuereinheit den perfundierten Fluidfluß so regelt, daß ein bestimmter Gefäßinnendruck während des Pumpens möglichst konstant gehalten wird.

Wird mit der erfindungsgemäßen Vorrichtung eine Vene retroinfundiert, dann ist die Leitung vorteilhaft mit einer Absaugeinrichtung für Blut aus der Vene des Patienten verbunden, wobei die Steuereinheit Signale vom Herzschlag des Patienten empfängt und Pump- und Saugintervalle definiert, die mit dem Herzzyklus des Patienten synchronisiert sind.

Gemäß einer bevorzugten Ausführungsform ist die Zufuhr- und Absaugleitung ein mehrläufiger Katheter. Bei einer zur Retroinfusion geeigneten Ausführungsform ist er ein mindestens vierläufiger Venenkatheter, wobei eine Zufuhrleitung für das Fluid, eine Absaugleitung für das abgesaugte Blut, eine Meßleitung zur Bestimmung des Veneninnendrucks und eine Steuerleitung für das vergrößerbare Dichtungsmittel vorgesehen sind. Zur Arterienperfusion wird vorteilhaft ein mindestens dreiläufiger Arterienkatheter verwendet, wobei eine Zufuhrleitung für das Fluid, eine Meßleitung zur Bestimmung des Arterieninnendrucks und eine Steuerleitung für das vergrößerbare Dichtungsmittel vorgesehen sind.

Das Dichtungsmittel ist vorteilhaft ein druckgesteuert aufblasbarer Ballon, so daß als Katheter ein Ballonkatheter verwendet werden kann. Der Ballon befindet sich dann bevorzugt an dem in das Blutgefäß eingeführten Ende des Katheters. Die Meßleitung zur Bestimmung des Gefäßinnendrucks kommuniziert an einem Ende mit dem Inneren des Gefäßes und weist an ihrem anderen Ende einen Drucksensor auf. Es ist aber auch möglich, einen Drucksensor am proximalen Ende der Leitung anzuordnen, der mit einem feinen Kabel, das beispielsweise durch die Steuerleitung für den aufblasbaren Ballon geführt sein kann, mit der Steuereinheit verbunden ist. In diesem Fall wäre beispielsweise ein dreiläufiger Katheter ausreichend. Zudem können Zufuhrleitung und Absaugleitung am proximalen Ende des Katheters als eine Leitung ausgeführt sein, die dann über ein schaltbares 3-Wege-Ventil mit dem Versorgungsreservoir oder dem Absaugreservoir verbunden wird.

Je nach Aufgabenstellung kann man auch einen Katheter vorsehen, der zusätzliche Leitungen, beispielsweise ein Glasfaserkabel für Laseranwendungen oder Videoaufzeichnungen enthält.

Bei einer bevorzugten Ausführungsform umfassen die Regelungsmittel für den perfundierten Fluidfluß einen Durchflußregler, der vorteilhaft so ausgebildet ist, daß die Zufuhrleitung im Bereich dieses Durchflußreglers einen elastisch nachgiebigen Schlauch aufweist, wobei der Durchflußregler ein von einem Elektromotor betriebenes Klemmorgan umfaßt, das den elastischen Schlauch mehr oder weniger stark zusammenpreßt und so den retroinfundierten Fluß des Fluids in das Blutgefäß steuert. Bevorzugt ist der Elektromotor ein von der Steuereinheit gesteuerter Schrittmotor, wobei ein auf der Achse des Schrittmotors angebrachter Exzenter das Klemmorgan betätigt, wobei das Klemmorgan bevorzugt als ein im wesentlichen zur Fluidzufuhrleitung senkrecht orientierter Querbalken ausgebildet ist. Dabei liegt die Zufuhrleitung auf einer starren Unterlage auf.

Bevorzugt ist das distale Ende der Zufuhrleitung mit einem unter Druck stehenden Reservoir für das Fluid verbunden, das vorteilhaft einen Druckaufnehmer zur Überwachung des Drucks in dem Reservoir besitzt. Aus diesem Reservoir wird die Zufuhrleitung mit dem zu infundierenden Fluid versorgt.

Bei einer weiteren Ausführungsform wird dieses Reservoir über eine Rollerpumpe mit dem aus einer Arterie des Patienten entnommenen Blut gespeist.

Die Absaugvorrichtung für das venöse Blut weist bevorzugt eine Unterdruckpumpe und ein Reservoir für das abgesaugte Blut auf. Will man das abgesaugte Blut dem Patienten wieder zuführen, so wird man dieses Blut bevorzugt in ein Reservoir leiten, aus dem es dann über eine Rollerpumpe, eine Luftfalle und eine Entschäumanordnung einer Vene des Patienten zugeführt werden kann.
Die Synchronisation mit dem Herzzyklus des Patienten wird bevorzugt über die Ableitung eines Elektrokardiogramms (EKG) durchgeführt, wobei besonders vorteilhaft die R-Zacke als Triggersignal verwendet wird. Der Pumpzyklus beginnt vorteilhaft zwischen 15 und 50% des R-R-Intervalls und endet mit dem Beginn der folgenden R-Zacke. Es ist aber auch denkbar, die Pumpphase in weiten Grenzen zu variieren, so daß es gegebenenfalls auch über die folgende R-Zacke des EKGs hinaus andauern kann. Üblicherweise wird eine festes Verhältnis von Pumpphasen zu Herzzyklen gewählt, beispielsweise 1:1, 1:2, 1:3 usw. Bevorzugt wird zwischen den einzelnen Pumpphasen immer abgesaugt. Es kann aber auch vorteilhaft sein, Phasen vorzusehen, in denen weder gepumpt noch gesaugt wird.

Die Vorrichtung zur Steuerung des Koronarvenendrucks während eines einzelnen Pumpintervalls weist gegenüber den bekannten Retroinfusionsvorrichtungen zahlreiche Vorteile auf:

Bei der arteriellen Perfusion, beispielsweise bei der antegraden Katheterperfusion einer Koronararterie, kann sowohl bei geblocktem als auch ungeblocktem Ballon an der Katheterspitze der gewünschte Perfusionsdruck aufrecht erhalten werden. Der Druck laßt sich in gewissen Toleranzgrenzen in einem engen Bereich um den optimalen Perfusionsdruck regeln und weitgehend konstant halten. Inbesondere ist es aber möglich, eine Überperfusion und die damit verbundenen hämorrhagischen Gewebeinfarkte auch dann zu vermeiden, wenn der Abstrom des Perfusats im Gewebe gestört ist. Weitere Perfusion von Fluid findet nach einer unzulässigen Erhöhung des arteriellen Drucks nämlich nur dann wieder statt, wenn der Druck wieder unter den vorgegebenen Sollwert gesunken ist.

Bei der Retroinfusion wird durch die Verhinderung einer wesentlichen Erhöhung des Koronarvenendrucks über einen zuvor eingestellten Grenzwert im Laufe eines Pumpintervalls verhindert, daß es zu potentiell gefährlichen Spitzendruckerhöhungen kommt, die Gefäßwandschädigungen und im Extremfall Gefäßrupturen verursachen könnten.

Der mit der erfindungsgemäßen Vorrichtung konstant einstellbare diastolische, venös-arterielle Druckgradient erlaubt eine äußerst wirksame Retroinfusion, da einerseits der optimale Druckgradient für eine nutritive Durchblutung vorhanden ist und andererseits eine Überperfusion vermieden wird.

Da der individuell unterschiedliche Koronarvenendruck, ab dem es zu einem vermehrten Abfluß von retroinfundiertem arteriellem Blut in die systematische Zirkulation kommt, mittels des Plateaudrucks bei Koronarvenenokklusion noch vor der Unterbrechung der antegraden Perfusion bestimmbar ist, ermöglicht die Erfindung die Einstellung des optimalen Koronarvenendruckbereichs für jeden Patienten.

Vor allem aber werden sowohl der Basisstoffwechsel als auch die regionale Myokardfunktion im Ischämiebereich gegenüber dem bekannten Verfahren deutlich verbessert. Dabei ist die Effektivität der Myokardprotektion insbesondere bei Patienten mit gering ausgeprägter arterieller Kollateralisierung deutlich erhöht. Eine Schädigung der retroinfundierten Venen kann mit dem erfindungsgemäßen Verfahren praktisch ausgeschlossen werden. Erste klinische Tests lassen vermuten, daß bei Angioplastien das Komplikationsrisiko verringert werden kann.

Die erfindungsgemäße Vorrichtung zur gefäßdruckgesteuerten selektiven Perfusion von Körpergefäßenv ist aber nicht nur auf Anwendungen bei der kurzzeitigen Ischämieprotektion begrenzt. Darüber hinaus sind länger andauernde Anwendungen der Methode, beispielsweise bei Komplikationen mit anhaltendem Verschluß der Koronararterie als Überbrückung bis zur operativen, notfallmäßigen Bypass-Versorgung möglich. Diese längerfristige Ischämieprotektion wird erst durch die hohe Effektivität der Gewebeversorgung mit dem erfindungsgemäßen Verfahren ermöglicht. Insbesondere, wenn das abgesaugte venöse Blut, gereinigt und entschäumt, in eine Körpervene des Patienten reinfundiert wird, ist auch eine längerfristige Anwendung des Retroinfusionsverfahrens bei Aufrechterhaltung des Funktionsstoffwechsels möglich.

Ein weiterer Anwendungsbereich liegt in der Identifizierung von chronischer, aber reversibler regionaler linksventrikulärer Dysfunktion, die auch als sogenanntes "hybernating myocardium" bezeichnet wird. Dabei ist in Ergänzung zu nuklearmedizinischen Methoden und NMR-Methoden eine Bestimmung des myokardialen Stoffwechsels möglich, die eine Differenzierung des nekrotischen oder vernarbten Myokardgewebes von noch potentiell stoffwechselaktiven Myokardzellen ermöglicht. Dabei kann, beispielsweise zur Abklärung des möglichen Erfolgs einer Bypass-Operation, über retrograde Perfusion festgestellt werden, ob und in welchem Umfang die Myokardfunktion durch Verbesserung der nutritiven Perfusion wieder hergestellt werden kann.

Als ein weiteres mögliches Anwendungsgebiet der Erfindung zeichnet sich bereits heute die Perfusion oder Reinfusion von in vitro gentherapeutisch behandelter Zellen in den Körper von Patienten ab.

Eine bevorzugte Ausführungsform der Erfindung wird im folgenden anhand der beigefügten Zeichnung näher erläutert. Das Ausführungsbeispiel behandelt eine Vorrichtung zur Retroinfusion von Körpervenen, ist aber im wesentlichen auch auf die Perfusion von Arterien übertragbar. Der wesenliche Unterschied besteht bei der Perfusion von Arterien darin, daß Blut nicht abgesaugt wird und demnach auch eine dreiläufiger Katheter eingesetzt werden kann.

In der Zeichnung zeigt:
- Fig. 1: eine schematische Darstellung einer erfindungsgemäßen Vorrichtung zur venendruckgesteuerten selektiven Absaugung und Retroinfusion von Körpervenen;
- Fig. 2: eine bevorzugte Ausführungsform des venenseitigen Endes der Retroinfusionsleitung der erfindungsgemäßen Vorrichtung, wobei die Leitung im vorliegenden Fall als vierläufiger Ballonkatheter ausgebildet ist;
- Fig. 3: einen Querschnitt entlang der Linie III-III des Katheters der Fig. 2;
- Fig. 4: eine Detaildarstellung einer bevorzugten Ausführungsform des Durchflußreglers der Vorrichtung der Fig. 1;
- Fig. 5: die schematische Darstellung einer Anwendung der vorliegenden Erfindung bei der Myokardprotektion bei Angioplastie;
- Fig. 6: Schaubilder des zeitlichen Verlaufs bestimmter Systemparameter.

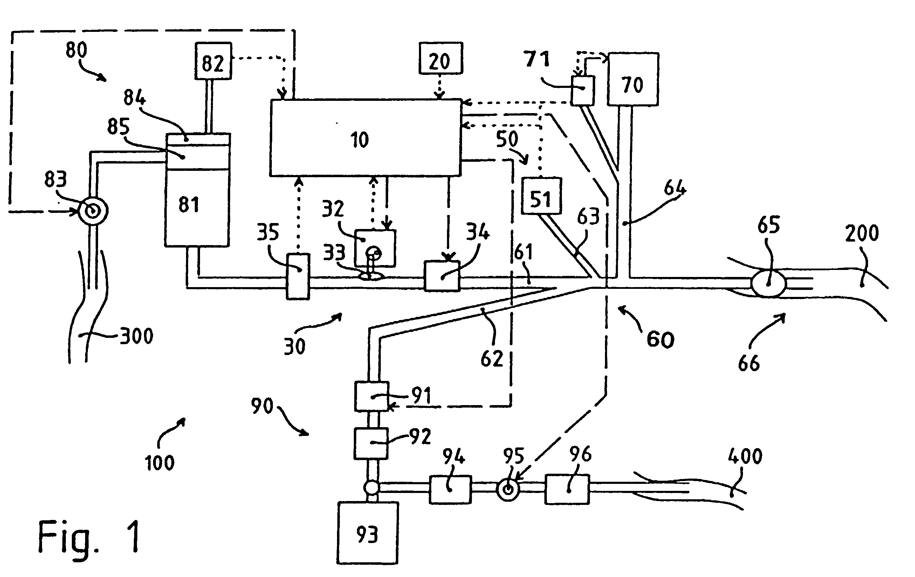

In Figur 1 ist eine bevorzugte Ausführungsform der erfindungsgemäßen Vorrichtung 100 zur venendruckgesteuerten, selektiven Absaugung und Retroinfusion von Körpervenen dargestellt. Die Vorrichtung weist eine, im vorliegenden Fall vierläufige Schlauchleitung 60 auf, die als Retroinfusionskatheter in die zu infundierende Körpervene 200 (beispielsweise die AIV-Vene) eingeführt wird. Am proximalen (venenseitigen) Schlauchende ist ein Dichtungsmittel 65 vorgesehen, bei dem es sich vorteilhaft um einen aufblasbaren Ballon handelt, der die Vene 200 gegenüber der Leitung 60 abdichtet, aber gleichzeitig den Durchtritt von Fluid aus der Schlauchleitung in die Vene oder umgekehrt ermöglicht. Zu diesem Zweck ist insbesondere eine Zufuhrleitung 61 vorgesehen, durch die Fluid in die Vene 200 gepumpt werden kann. Dazu ist die Zufuhrleitung an ihrem distalen (dem venenseitigen Ende gegenüberliegenden) Ende mit einer Fluidversorgung 80 verbunden. Die Fluidversorgung 80 umfaßt ein unter Druck stehendes Fluidreservoir 81, wobei der Druck im Reservoir mittels eines Druckaufnehmers 82 überwacht wird. Wenn es sich bei dem zu retroinfundierenden Fluid um körpereigenes Blut des Patienten handelt, kann das Reservoir 81 über eine Pumpe 83 mit einer Körperarterie 300 (z. B. mit der Arteria femoralis) des Patienten verbunden sein. In diesem Fall wird Blut aus der Arterie 300 abgesaugt und gegebenenfalls über eine Luftfalle und/oder Entschäumer 84 und ein Blutfilter 85 in das Reservoir 81 geleitet. An der Zufuhrleitung 61 sind Mittel 30 zur Regelung des Fluidflusses vorgesehen. Zu diesem Durchflußregler 30 gehört insbesondere ein Verschlußventil 34, mit dessen Hilfe die Verbindung zwischen Reservoir 81 und Vene 200 vollständig unterbrochen werden kann. In einer besonders einfachen Ausführungsform des Durchflußreglers ist die Zufuhrleitung 61 im Bereich des Reglers als elastisch nachgiebiger Schlauch ausgebildet, wobei der Durchflußregler 30 ein auf diesen Schlauch wirkendes Klemmorgan 31 umfaßt, das über einen Elektromotor, bevorzugt einen Schrittmotor 32, betätigt wird. Durch mehr oder weniger starkes Abquetschen der Schlauchleitung wird der retroinfundierte Fluidfluß reguliert. Als Maß für den zu infundierenden Fluidfluß dient der intravenöse Druck in der Körpervene 200. Hierzu weist der Retroinfusionskatheter 60 eine Meßleitung 63 auf, die eine kommunizierende Verbindung zwischen einem am distalen Ende der Meßleitung 63 angeordneten Drucksensor 51 und dem Veneninneren herstellt. Der Drucksensor kann aber auch als proximaler Drucksensor 52 am venenseitigen Ende 66 der Schlauchleitung 60 vorgesehen sein. In diesem Fall kann auch ein dreiläufiger Katheter verwendet werden, wobei die elektrische Verbindung des Drucksensors 52 mit den Mitteln zur Messung des Venendrucks 50 durch eines der übrigen Lumen des Katheters geführt werden kann.

Die so gemessenen intravenösen Druckwerte werden von einer Steuereinheit 10 geregelt, die über den Schrittmotor 32 des Durchflußreglers 30 das Klemmorgan entsprechend einstellt. An der Zufuhrleitung 61 kann außerdem ein Ultraschallmeßkopf 35 vorgesehen sein, der einerseits zur Detektion von Luftblasen in dem zu retroinfundierenden Fluid und andererseits zur Bestimmung des Fluidflusses selbst dient. Hierzu weist der Ultraschallmeßkopf 35 einen Ultraschallsender und einen Ultraschallempfänger auf, wobei das reflektierte Ultraschallsignal zur Detektion von Luftblasen und das Dopplerverschobene Ultraschallsignal zur Bestimmung des Fluidflusses herangezogen wird.

Ein weiteres Lumen des Katheters ist eine Absaugleitung 62, die zum Absaugen von Blut oder Retroinfusat aus der Vene 200 des Patienten dient. Die Absaugeinrichtung 90 umfaßt ein Verschlußventil 91, eine Unterdruckpumpe 92 und einen Behälter 93 zum Auffangen des abgesaugten Fluids. Gegebenenfalls kann vorgesehen sein, das abgesaugte Fluid nicht aufzufangen und zu verwerfen, sondern es gereinigt dem Patienten wieder zuzuführen. Dies ist besonders bei längerfristigen Anwendungen der erfindungsgemäßen Vorrichtung sinnvoll. Hierzu ist ein Zwischenreservoir 94 vorgesehen, wobei das dort aufgefangene Blut über eine Rollerpumpe 95, und eine Luftfalle 96 und gegebenenfalls ein Blutfilter dem Patienten in eine andere Vene 400 wieder zugeführt wird.

Für eine effektive Retroinfusion ist es wichtig, daß die zu infundierende Vene 200 stromaufwärts, in Infusionsrichtung gesehen, dicht verschlossen ist, so daß das Retroinfusat ausschließlich in den zu versorgenden Gewebebereich fließt. Hierzu ist am proximalen Ende 66 des Katheters 60 als Dichtungsmittel 65 ein pneumatisch aufblasbarer Ballon, vorgesehen. Ein viertes Lumen des Katheters bildet daher die Steuerleitung 64, die sich nicht in die Vene 200 öffnet, sondern den Ballon 65 mit einer druckgesteuerten Ballonpumpe 70 verbindet. Zur Drucksteuerung ist ein Drucksensor 71 für den Ballondruck an der Leitung 64 vorgesehen. Wenn der Katheter in die zu retroinfundierende Vene eingeführt ist, kann der Ballon 65 durch Einpumpen von Luft, gegebenenfalls aber auch durch Einpumpen einer Flüssigkeit, aufgeblasen werden. Er verschließt dann die Vene am proximalen Ende der Schlauchleitung 60 dicht, wobei aber die kommunizierende Verbindung zwischen Zufuhrleitung 61, Absaugleitung 62 und Druckmeßleitung 63 mit dem Inneren der Vene 200 gewährleistet bleibt.

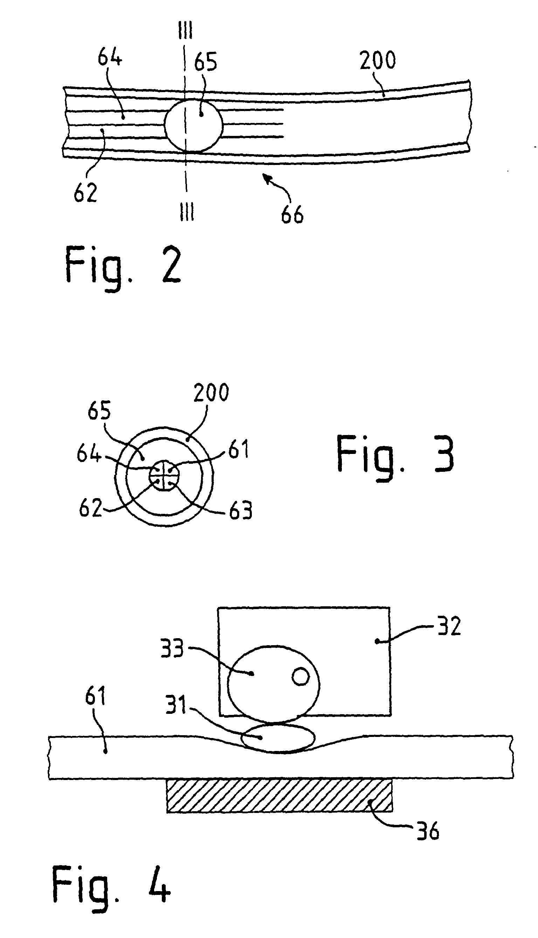

In Figur 2 ist das in die Vene eingeführte, proximale Ende des Retroinfusionskatheters 60 der in Figur 1 dargestellten Retroinfusionsvorrichtung 100 vergrößert dargestellt. Die einzelnen Leitungen 61, 62, 63, 64 des vierläufigen Katheters 60 sind in dem in den Patienten einführbaren Abschnitt des Katheters zusammen verschweißt und trennen sich erst außerhalb des Patienten in Einzelleitungen. Dabei muß beispielsweise die Zufuhrleitung 61 keine durchgehende Leitung sein, sondern kann, außerhalb des Patienten, über ein nicht dargestelltes Kupplungsstück mit einer weiteren Leitung zum Versorgungsreservoir 61 verbunden sein.

Figur 3 zeigt einen Querschnitt entlang der Linie III-III des proximalen Endes des Katheters der Figur 2. Der Ballon ist dabei in der aufgeblasenen Stellung dargestellt und verschließt den Bereich zwischen Leitung 60 und Vene 200 dicht.

Figur 4 zeigt eine vergrößerte Detaildarstellung einer bevorzugten Ausführungsform des Durchflußreglers der Vorrichtung der Figur 1. Die Zufuhrleitung 61 besteht in diesem Bereich aus einem elastischen Schlauch und wird von einem als Querbalken ausgebildeten Klemmorgan 31 zusammengepreßt. Der Querbalken wird von einem Schrittmotor 32 betätigt, auf dessen Welle eine exzentrische Steuerscheibe vorgesehen ist, die auf den Querbalken 31 einwirkt. Die Taktfrequenz des Schrittmotors ist dabei so gewählt, daß in weniger als 25 ms ein neuer Fluidfluß in der Zufuhrleitung 61 eingestellt werden kann. Die entsprechende Steuerung erfolgt über die Steuereinheit 10, die aus dem jeweils gemessenen Druck und insbesondere dem momentanen Druckverlauf die erforderliche Stellung des Klemmorgans berechnet und entsprechende Instruktionen an den Schrittmotor schickt. Die Leitung 61 ruht dabei auf einem Gegenlager 36, das eine Bewegung der Leitung beim Zusammenquetschen verhindert.

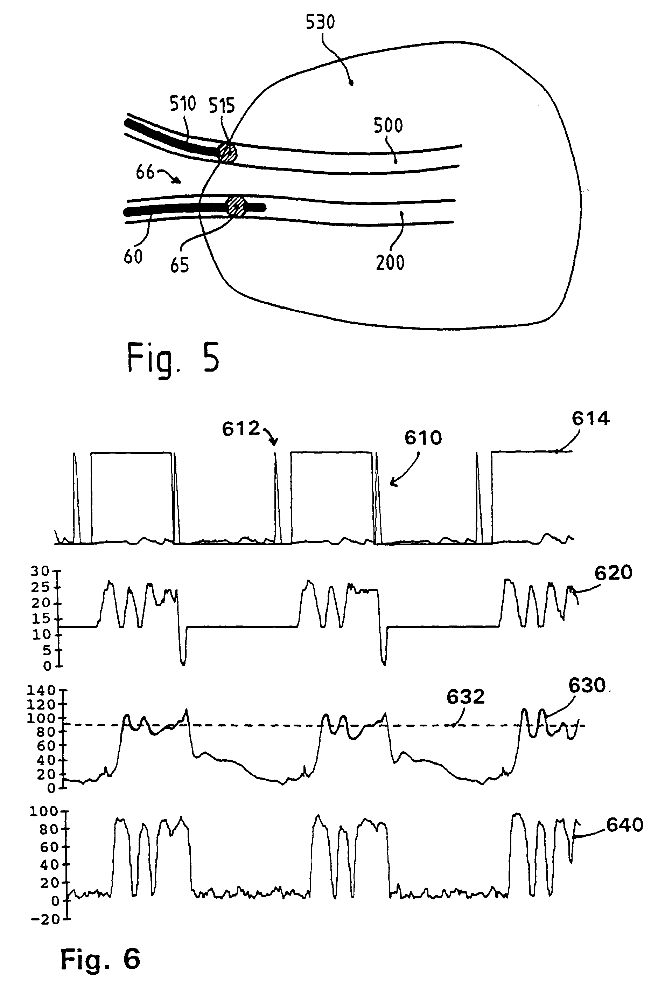

In Figur 5 ist schematisch eine typische Anwendung der vorliegenden Erfindung dargestellt. Es handelt sich dabei um den Einsatz bei der Myokardprotektion während einer Angioplastie. Dabei wird eine durch arteriosklerotische Plaque verengte Koronararterie 500 mittels eines Ballonkatheters 510 erweitert. Solange der Ballon 515 des Ballonkatheters aufgeblasen ist, wird der von dieser Arterie normalerweise versorgte Bereich des Myokard 530 nicht mehr ausreichend mit Sauerstoff und Nährstoffen versorgt. Zu diesem Zweck wird in eine diesen Myokardbereich drainierende Vene 200 ein Retroinfusionskatheter 60 der erfindungsgemäßen Vorrichtung unter Röntgenkontrolle eingeschoben. Aus einer anderen Arterie des Patienten entnommenes sauerstoff- und nährstoffhaltiges Blut wird durch die Leitung 60 in das Ischämiegebiet retroinfundiert, so daß eine funktionelle Beeinträchtigung dieses Gebietes verhindert wird.

Zur Durchführung der Myokardprotektion wird die zu retroinfundierende Vene des Patienten in Abhängigkeit von dem in Betracht kommenden Myokardbereich ausgewählt und der Retroinfusionskatheter 60 der erfindungsgemäßen Vorrichtung 100 vorzugsweise unter Röntgenkontrolle in die Nähe des zu schützenden Myokardbereichs vorgeschoben. Je nachdem ob venöser Blutfluß vorhanden ist oder nicht, wird der intravenöse Druck bestimmt oder eine mit jedem Pumpintervall höher werdende Fluidmenge infundiert und der sich dabei einstellende Plateaudruck gemessen. Daraus läßt sich der gewünschte Sollwert des intravenösen Drucks während der Retroinfusionsintervalle bestimmen. Vorzugsweise entspricht der der Sollwert dem gewünschten Plateaudruck, er kann aber auch etwas höher oder niedriger gewählt werden.

Die Retroinfusion mit Blut oder einem anderen Fluid wird mit dem Herzschlag des Patienten synchronisiert. Dazu wird beispielsweise ein Elektrokardiogramm mit einem EKG-Gerät 20 (siehe Figur 1) abgeleitet. In Figur 6 ist der zeitliche Verlauf einiger wichtiger Systemparameter dargestellt. Die Steuereinheit 10 wertet die R-Zacke 612 der EKG-Ableitung 610 aus. Mit dieser R-Zacke des Herzzyklusses werden die Retroinfusions- bzw. Absaugphasen synchronisiert. Die Linie 614 zeigt die Triggerphase für das Retroinfundieren von Fluid. Diese Phase beginnt bevorzugt nach 15 bis 50 % eines R-R-Intervalls. Die Linie 620 zeigt die momentane Stellung des Durchflußreglers, wobei Änderungen nach oben eine Erhöhung und Änderungen nach unten eine Erniedrigung des Durchflusses bedeuten. Die Kurve 630 zeigt den aktuellen Druck in der zu retroinfundierenden Vene, wobei die horizontale Linie 632 den vorgewählten Solldruck darstellt. Die Einheit des Drucks ist hier mmHg.

Die Linie 640 zeigt das retroinfundierte Fluidvolumen in ml/min. Wie in der Zeichnung dargestellt, ist es möglich, den gewünschten intravenösen Solldruck gut einzuhalten.

Zwischen den Triggerphasen wird Blut bzw. Retroinfusat aus der Vene 200 abgesaugt.

Typischerweise werden pro Pumpintervall ca. 0,5 bis 1,5 ml Fluid infundiert. Damit liegt die durchschnittliche Pumpmenge zwischen 30 und 150 ml/min. Diese Werte werden aber von dem verwendeten Kathetervolumen, der Katheterlänge und dem Vordruck im zufuhrseitigen Hochdrucksystem mitbeeinflußt.

Typische Retroinfusionsdrucke (Sollwerte) liegen je nach Patient und retroinfundierter Vene zwischen 30 und 110 mmHg.

## Patentansprüche

1. Vorrichtung zur gefäßdruckgesteuerten selektiven Perfusion von Körpergefäßen mit einem Fluid,
mit einer in ein Körpergefäß (200) eines Patienten einbringbaren, am proximalen Ende offenen Schlauchleitung (60), die mit einem unter Druck stehenden Fluid beschickbar ist, das in das Körpergefäß (200) gepumpt werden soll,
wobei am proximalen Ende ein vergrößerbares Dichtungsmittel (65) vorgesehen ist, mit dem das Gefäß (200) gegenüber der Leitung (60) abdichtbar ist, und
mit Mitteln (50) zur Messung des Gefäßinnendrucks,
wobei außerdem Regelungsmittel (30) vorgesehen sind, mit deren Hilfe eine Steuereinheit (10) den perfundierten Fluidfluß so regelt, daß ein bestimmter Sollwert des Gefäßinnendrucks während des Pumpens möglichst konstant gehalten wird.

2. Vorrichtung gemäß Anspruch 1, **dadurch gekennzeichnet, daß** die Leitung (60) mit einer Absaugeinrichtung (90) für Blut aus einer Vene (200) des Patienten verbunden ist, und daß die Steuereinheit (10) Signale vom Herzschlag des Patienten empfangen kann und Pump- und Saugintervalle definieren kann, die mit dem Herzzyklus des Patienten synchronisiert sind.

3. Vorrichtung gemäß einem der Ansprüche 1 oder 2, **dadurch gekennzeichnet, daß** die Leitung (60) ein mehrläufiger Katheter ist, insbesondere ein mindestens vierläufiger Venenkatheter zur Retroinfusion, wobei eine Zufuhrleitung (61) für das Fluid, eine Absaugleitung (62) für das abgesaugte Blut, eine Meßleitung (63) zur Bestimmung des Gefäßinnendrucks und eine Steuerleitung (64) für das vergrößerbare Dichtungsmittel (65) vorgesehen sind.

4. Vorrichtung gemäß Anspruch 1, **dadurch gekennzeichnet, daß** die Leitung (60) ein mehrläufiger Katheter ist, insbesondere ein mindestens dreiläufiger Arterienkatheter zur Perfusion, wobei eine Zufuhrleitung (61) für das Fluid, eine Meßleitung (63) zur Bestimmung des Gefäßinnendrucks und eine Steuerleitung (64) für das vergrößerbare Dichtungsmittel (65) vorgesehen sind.

5. Vorrichtung gemäß einem der Ansprüche 1 bis 4, **dadurch gekennzeichnet, daß** das Dichtungsmittel (65) ein druckgesteuert aufblasbarer Ballon ist.

6. Vorrichtung gemäß einem der Ansprüche 4 oder Anspruch 5 in Verbindung mit Anspruch 3 oder 4, **dadurch gekennzeichnet, daß** die Meßleitung (63) an einem Ende mit dem Gefäßinneren kommuniziert und an ihrem anderen Ende einen Drucksensor (51) aufweist.

7. Vorrichtung gemäß einem der Ansprüche 1 bis 5, **dadurch gekennzeichnet, daß** die Mittel (50) zur Messung des Gefäßinnendrucks einen am proximalen Ende der Leitung (60) angeordneten Drucksensor (52) umfassen, der mit der Steuereinheit (10) verbunden ist.

8. Vorrichtung gemäß einem der Ansprüche 1 bis 7, **dadurch gekennzeichnet, daß** die Regelungsmittel (30) für den perfundierten Fluidfluß einen mit der Leitung (60) zusammenwirkenden Durchflußregler umfassen.

9. Vorrichtung gemäß Anspruch 8 in Verbindung mit Anspruch 3 oder 4, **dadurch gekennzeichnet, daß** die Zufuhrleitung (60,61) im Bereich des Durchflußreglers (30) von einem elastisch nachgiebigen Schlauch gebildet wird, wobei der Durchflußregler (30) ein von einem Elektromotor betriebenes Klemmorgan (31) umfaßt, das den elastischen Schlauch mehr oder weniger stark zusammenpreßt und so den Fluß des Fluids in das Gefäß steuert.

10. Vorrichtung gemäß einem der Ansprüche 1 bis 9 in Verbindung mit Anspruch 3 oder 4, **dadurch gekennzeichnet, daß** das distale Ende der Zufuhrleitung (60,61) mit einem unter Druck stehenden Reservoir (81) für das Fluid verbunden ist, wobei ein Druckaufnehmer (82) zur Überwachung des Drucks in dem Reservoir (81) vorgesehen ist.

11. Vorrichtung gemäß Anspruch 10, **dadurch gekennzeichnet, daß** das Reservoir (81) über eine Rollerpumpe (83) mit dem aus einer Arterie (300) des Patienten entnommenen Blut gespeist werden kann.

12. Vorrichtung gemäß einem der Ansprüche 2 bis 11 in Verbindung mit Anspruch 2, **dadurch gekennzeichnet, daß** die Absaugeinrichtung (90) eine Unterdruckpumpe (92) und einen Behälter (93) für das abgesaugte Blut umfaßt.

13. Vorrichtung gemäß einem der Ansprüche 2 bis 12 in Verbindung mit Anspruch 2, **dadurch gekennzeichnet, daß** die Absaugeinrichtung (90), ein Reservoir (94), eine Rollerpumpe (95) und eine Luftfalle (96) miteinander verbunden sind, um einer Vene (400) des Patienten das abgesaugte Blut gereinigt zuführen zu können.

## Claims

1. Device for the selective perfusion of a fluid through vessels of the body, controlled by the pressure in the vessels,
with a tubing line (60) which can be introduced into a patient's body vessel (200), which tubing line is open at the proximal end and can be charged with a fluid under pressure which is to be pumped into the body vessel (200),
the proximal end being provided with an enlargeable sealing means (65) which seals off the vessel (200) from the line (60), and
with means (50) for measuring the internal pressure of the vessel,
wherein regulating means (30) are additionally provided by means of which a control unit (10) controls the flow of perfused fluid such that a predefined pressure inside the vessel is maintained as constant as possible during pumping.

2. Device according to Claim 1, **characterized in that** the line (60) is connected to a suction device (90) for blood from a patient's vein (200), and a control unit (10) is provided which is responsive to signals from the patient's heartbeat and which can define pumping and suction intervals which are synchronized with the patient's heart cycle.

3. Device according to one of Claims 1 and 2, **characterized in that** the line (60) is a multi-lumen catheter, preferably an at least four-lumen vein catheter for retroinfusion, including an admission line (61) for the fluid, a suction line (62) for the suctioned blood, a measurement line (63) for determining the internal pressure of the vessel, and a control line (64) for the enlargeable sealing means (65).

4. Device according to Claim 1, **characterized in that** the line (60) is a multi-lumen catheter, preferably an at least three-lumen artery catheter for perfusion, including an admission line (61) for the fluid, a measurement line (63) for determining the internal pressure of the artery, and a control line (64) for the enlargeable sealing means (65).

5. Device according to one of Claims 1 to 4, **characterized in that** the sealing means (65) is a pressure-controlled, inflatable balloon.

6. Device according to one of Claims 4 or Claim 5 in connection with Claim 3 or 4, **characterized in that** the measurement line (63) communicates at one end with the inside of the vessel and has a pressure sensor (51) at its other end.

7. Device according to one of Claims 1 to 5, **characterized in that** the means (50) for measuring the vessel pressure comprise a pressure sensor (52) arranged at the proximal end of the line (60), which pressure sensor (52) is connected to the control unit (10).

8. Device according to one of Claims 1 to 7, **characterized in that** the control means (30) for the perfused fluid flow comprises a flow control which cooperates with line (60).

9. Device according to Claim 8 in connection with Claim 3 or 4, **characterized in that** the admission line (60, 61) is formed by an elastically yielding tubing in the area of the flow control (30), the flow control (30) comprising a clamping member (31) which is driven by an electric motor and which presses the elastic tubing together to a greater or lesser extent and so controls the flow of the fluid into the vessel.

10. Device according to one of Claims 1 to 9 in connection with Claim 3 or 4, **characterized in that** the distal end of the admission line (60, 61) is connected to a fluid reservoir (81) which is under pressure and which has a pressure sensor (82) for monitoring the pressure in the reservoir (81).

11. Device according to Claim 10, **characterized in that** the blood taken from an artery (300) of the patient can be fed to the reservoir (81) via a roller pump (83).

12. Device according to one of Claims 2 to 11 in connection with claim 2, **characterized in that** the suction device (90) has a vacuum pump (92) and a reservoir (93) for the suctioned blood.

13. Device according to one of Claims 2 to 12 in connection with claim 2, **characterized in that** the suction device (90), a reservoir (94), a roller pump (95) and an air trap (96) are connected to one another in order to allow for the delivery of the suctioned blood, cleaned, to a vein (400) of the patient.

## Revendications

1. Dispositif permettant d'injecter un fluide dans des vaisseaux sanguins par perfusion sélective régulée par la pression vasculaire,
comprenant une conduite en tuyaux souples (60) ouverte à l'extrémité proximale pouvant être introduite dans un vaisseau sanguin (200) d'un patient, qui peut être chargée par un fluide sous pression qui doit être pompé dans le vaisseau sanguin (200),
dans lequel un dispositif d'obturation pouvant être agrandi (65) est prévu à l'extrémité proximale, grâce auquel le vaisseau (200) peut être étanchéifié par rapport à la conduite (60), et
comprenant des moyens (50) pour mesurer la pression vasculaire interne,
des moyens de régulation (30) étant également prévus, à l'aide desquels une unité de commande (10) régule l'écoulement du fluide de la perfusion de telle sorte qu'une valeur de consigne déterminée de la pression vasculaire interne soit maintenue aussi constante que possible pendant le pompage.

2. Dispositif selon la revendication 1, **caractérisé en ce que** la conduite (60) est reliée à un dispositif d'aspiration (90) pour le sang d'un vaisseau (200) du patient, et **en ce que** l'unité de commande (10) peut recevoir des signaux du rythme cardiaque du patient et peut définir des intervalles de pompage et d'aspiration qui sont synchronisés avec le cycle cardiaque du patient.

3. Dispositif selon l'une des revendications 1 ou 2, **caractérisé en ce que** la conduite (60) est un cathéter à voies multiples, en particulier un cathéter veineux à au moins quatre voies pour rétro-infusion, dans lequel sont prévues une conduite d'alimentation (61) pour le fluide, une conduite d'aspiration (62) pour le sang aspiré, une conduite de mesure (63) pour la détermination de la pression vasculaire interne et une conduite de commande (64) pour le dispositif d'obturation pouvant être agrandi (65).

4. Dispositif selon la revendication 1, **caractérisé en ce que** la conduite (60) est un cathéter à voies multiples, en particulier un cathéter artériel à au moins trois voies pour perfusion, dans lequel sont prévues une conduite d'alimentation (61) pour le fluide, une conduite de mesure (63) pour la détermination de la pression vasculaire interne et une conduite de commande (64) pour le dispositif d'obturation pouvant être agrandi (65).

5. Dispositif selon l'une quelconque des revendications 1 à 4, **caractérisé en ce que** le dispositif d'obturation (65) est un ballon gonflable régulé par la pression.

6. Dispositif selon l'une des revendications 4 ou 5 conjointement avec l'une des revendications 3 ou 4, **caractérisé en ce que** la conduite de mesure (63) communique avec l'intérieur du vaisseau sanguin à l'une de ses extrémités et présente un capteur de pression (51) à son autre extrémité.

7. Dispositif selon l'une quelconque des revendications 1 à 5, **caractérisé en ce que** les moyens (50) pour mesurer la pression vasculaire interne comprennent un capteur de pression (52) disposé à l'extrémité proximale de la conduite (60), lequel est relié à l'unité de commande (10).

8. Dispositif selon l'une quelconque des revendications 1 à 7, **caractérisé en ce que** les moyens de régulation (30) pour l'écoulement du fluide de la perfusion comprennent un régulateur d'écoulement agissant conjointement avec la conduite (60).

9. Dispositif selon la revendication 8 conjointement avec l'une des revendications 3 ou 4, **caractérisé en ce que** la conduite d'alimentation (60, 61) est formée, dans la zone du régulateur d'écoulement (30), par une conduite élastique souple, le régulateur d'écoulement (30) comprenant un organe de serrage (31) commandé par un moteur électrique, qui comprime plus ou moins fort la conduite élastique et qui régule ainsi l'écoulement du fluide dans le vaisseau.

10. Dispositif selon l'une quelconque des revendications 1 à 9 conjointement avec l'une des revendications 3 ou 4, **caractérisé en ce que** l'extrémité distale de la conduite d'alimentation (60, 61) est reliée à un réservoir sous pression (81) pour le fluide, un capteur de pression (82) étant prévu pour la surveillance de la pression dans le réservoir (81).

11. Dispositif selon la revendication 10, **caractérisé en ce que** le réservoir (81) peut être alimenté par le sang prélevé d'une artère (300) du patient à l'aide d'une pompe à roulement (83).

12. Dispositif selon l'une quelconque des revendications 2 à 11 conjointement avec la revendication 2, **caractérisé en ce que** le dispositif d'aspiration (90) comprend une pompe de sous-pression (92) et un récipient (93) pour le sang aspiré.

13. Dispositif selon l'une quelconque des revendications 2 à 12 conjointement avec la revendication 2, **caractérisé en ce que** le dispositif d'aspiration (90), un réservoir (94), une pompe à roulement (95) et un piège à air (96) sont reliés entre eux pour pouvoir amener le sang aspiré purifié à une veine (400) du patient.
